# EUROPÄISCHE PATENTSCHRIFT

(11) **EP 2 794 553 B1**
(45) Veröffentlichungstag und Bekanntmachung des Hinweises auf die Patenterteilung: **05.09.2018**
(21) Anmeldenummer: 12790876.2
(22) Anmeldetag: 20.11.2012
(51) Int. Cl.: C07C 231/02, C07C 233/09

(54) **VERFAHREN ZUR HERSTELLUNG VON N-ALKYL(METH)ACRYLAMIDEN**
PROCESS FOR PREPARING N-ALKYL(METH)ACRYLAMIDES
PROCÉDÉ DE PRÉPARATION DE N-ALKYL(MÉTH)ACRYLAMIDES

(30) Priorität: 21.12.2011 DE 102011089363
(43) Veröffentlichungstag der Anmeldung: 29.10.2014
(73) Patentinhaber: Evonik Röhm GmbH, 64293 Darmstadt (DE)
(72) Erfinder: BRÖLL, Dirk, 63225 Langen (DE); LAUX, Benedikt, 55234 Monzernheim (DE); MAUL, Christian, 67435 Neustadt a. d. W. (DE); PETER, Patrick, 64289 Darmstadt (DE); ZIMMER, Claus, 67549 Worms (DE)
(86) Internationale Anmeldenummer: PCT/EP2012/073115
(87) Internationale Veröffentlichungsnummer: WO 2013/092076

(56) Entgegenhaltungen:
- WO-A1-2010/072480
- WO-A2-2010/021956

## Beschreibung

Die Erfindung betrifft ein Verfahren zur Herstellung von N-Alkyl(meth)acrylamiden durch Umsetzung von (Meth)acrylsäureanhydrid mit entsprechenden Alkylaminen.

Die Herstellung von N-Alkyl(meth)acrylamiden ist bekannt. Seit langem dient die Ritter-Reaktion zur Herstellung von Amiden aus Nitrilen und Substraten, die Carbeniumionen bilden können, wie. z.B. tertiäre oder sekundäre Alkohole in Gegenwart von starken Mineralsäuren. Die DE 3131096 nutzt dieses Verfahren. Nachteilig ist, dass die auch als Lösungsmittel fungierende Säure neutralisiert werden muss, und so große Salzmengen anfallen. Ferner enthält das Produkt störende Verunreinigungen wie z.B. Methacrylamid.

DE 4027843 beschreibt ein kontinuierliches Verfahren zur Herstellung N-substituierter (Meth)acrylsäureamide durch Umesterung von Alkylestern der (Meth)acrylsäure mit aliphatischen und aromatischen Aminen. Die Reaktion kommt zwar ohne Katalysator aus, erfolgt jedoch unter drastischen Bedingungen bei > 150° und einem Druck von ca. 160 bar.

Das in der WO 2010/072480 beanspruchte Verfahren zur Herstellung von N-Isopropylmethacrylamid durch Umsetzung von Methacrylsäureanhydrid mit Isopropylamin erfordert in der bevorzugten Variante die Anwesenheit eines inerten organischen Lösungsmittels, was mit Reinigungsaufwand zur Rezyklierung verbunden ist. In der lösungsmittelfreien Variante treten massive Wärmetönungen auf, die zu Verfärbungen des Produktes führen können.

WO 2010/021956 offenbart die Herstellung von N-Alkyl(alkyl)acrylamiden ebenfalls ausgehend vom entsprechenden Säureanhydrid und Alkylamin. Hier wird die Zugabe des Anhydrids zum vorgelegten Amin beansprucht. Diese Verfahrensvariante ist ebenfalls mir starker Wärmetönung verbunden, welche die bereits zitierten Verfärbungen verursachen kann. Darüber hinaus besteht nur ein enges Verfahrensfenster, in dem keine schwer störenden Verklumpungen auftreten. Aufgabe der vorliegen Erfindung ist es daher, ein alternatives Verfahren zur Herstellung von N-Alkyl(meth)acrylamiden zur Verfügung zu stellen, das die oben genannten Nachteile nicht aufweist und insbesondere Produkte in hoher Ausbeute und Reinheit erzeugt, die unter moderaten Reaktionsbedingungen, vorzugsweise 1 bis 10 bar und 20 bis 100 °C, und störungsfreiem Ablauf anfallen.

Gelöst werden diese sowie weitere im einzelnen nicht näher ausgeführte, jedoch aus der einleitenden Erörterung des Standes der Technik ohne weiteres erschließbare oder ableitbare Aufgaben durch ein Verfahren mit den Merkmalen des Patentanspruchs 1. Vorteilhafte Abwandlungen des erfindungsgemäßen Verfahrens werden in den auf Anspruch 1 rückbezogenen Ansprüchen unter Schutz gestellt.

Die Erfindung betrifft ein Verfahren zur Herstellung von N-Alkyl(meth)acrylamiden der allgemeinen Formel (1), 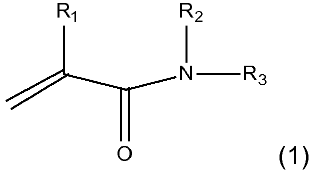 wobei R₁ für eine H- oder CH₃-Gruppe, R₂ und R₃ für Wasserstoff oder einen linearen, verzweigten oder cyclischen Alkylrest oder Arylrest mit 1 bis 12 C-Atomen steht. Die Umsetzung erfolgt indem man vorgelegtes (Meth)acrylsäureanhydrid mit einer wässrigen Lösung eines Alkylamins der Formel (2),

R₂R₃NH (2)

wobei R₂ und R₃ die oben angegebene Bedeutung besitzen, umsetzt und das entstehende Amid abtrennt. Die Bezeichnung (Meth)acrylsäureanhydrid bedeutet sowohl Methacrylsäureanhydrid als auch Acrylsäureanhydrid. Überraschend wurde gefunden, dass diese Vorgehensweise im Gegensatz zu WO 2010/021956 zu keinem Zeitpunkt zu unkontrollierten Niederschlägen in Form von Verklumpungen oder Ablagerungen an der Reaktorwand führt.

Als primäre Amine kommen primäre ggf. substituierte aliphatische Amine in Frage, wie beispielsweise Methylamin, Ethylamin, Propylamin, Butylamin, Pentylamin, Hexylamin, Heptylamin, Octylamin, Dodecylamin, Isopropylamin, Isobutylamin und Benzylamin sowie Allylamin und Ammoniak. Ferner können primäre cycloaliphatische Amine eingesetzt werden, wie beispielsweise Cyclopropylamin, Cyclobutylamin, Cyclopentylamin und Cyclohexylamin. Als primäre aromatische Amine können Anilin, die isomeren Aminotoluole, einzeln oder in Mischungen und die isomeren Xylidine, einzeln oder in Mischungen eingesetzt werden. Diese Verbindungen können gegebenenfalls durch ein oder mehrere Halogene substituiert sein.

Als sekundäre Amine können ebenfalls aliphatische, cycloaliphatische oder aromatische Amine eingesetzt werden, die gegebenenfalls durch ein oder mehrere Halogene substituiert sein können. Besonders bevorzugt sind Dimethylamin, Diethylamin, Methylethylamin, Dibutylamin, Dibenzylamin, Morpholin und gegebenenfalls substituierte Piperidine.

Da es sich hier um die Herstellung polymerisierbarer Verbindungen handelt, muss die Umsetzung in Gegenwart von Polymerisationsinhibitoren erfolgen. Die Reaktion kann unter Luftsauerstoff oder Schutzgas erfolgen. Im ersten Fall sind entsprechende Explosionsschutzgrenzen einzuhalten und unter Luftsauerstoff wirkende Inhibitoren einzusetzen. Da dass erfindungsgemäße Verfahren bevorzugt unter Schutzgas, besonders bevorzugt unter Stickstoff verläuft, können in diesem Fall nur Polymerisationsinhibitoren verwendet werden, die auch unter Ausschluss von Sauerstoff wirken. Als solche Polymerisationsinhibitoren sind z.B. 4-Hydroxy-2,2,6,6- tetramethylpiperidin-1-oxyl, N,N'-Diphenyl-p-phenylendiamin, Triazine, Phenazine, Phenothiazine, Methylenblau oder sterisch gehinderte Phenole, in der Fachwelt weithin bekannt. Diese Verbindungen können einzeln oder in Form von Mischungen eingesetzt werden und sind im Allgemeinen kommerziell erhältlich. Für weitere Details wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxidantien" und die an dieser Stelle zitierten Literaturstellen verwiesen. Besonders bevorzugt sind 4-Hydroxy-2,2,6,6- tetramethylpiperidin-1-oxyl oder 4-Methyl-2,6-di-tert.-butylphenol einzeln oder in Mischungen. Übliche Konzentrationen werden hier zwischen 1 bis 2000 ppm, bevorzugt 20 bis 200 ppm, besonders bevorzugt 40 bis 80 ppm, bezogen auf die Gesamtreaktionsmischung, eingesetzt.

Im erfindungsgemäßen Verfahren wird das (Meth)acrylsäureanhydrid in einem geeigneten Rührreaktor, gegebenenfalls mit aussenliegendem Wärmetauscher und Zirkulationspumpe, vorgelegt, mit Polymerisationsinhibitoren versetzt und auf unter 30 °C, bevorzugt auf 10 bis -5 °C abgekühlt. Der Reaktor wird mit Schutzgas, bevorzugt Stickstoff gespült. Die wässrige Lösung des Amins wird in einer Vorlage bereitgestellt und ebenfalls auf unter 30 °C, bevorzugt auf 10 bis -5 °C abgekühlt. Die Konzentration des Amins in der wässrigen Lösung kann 50 bis 90 %, bevorzugt 60 bis 80 %, besonders bevorzugt 65 bis 75% betragen. Das Molverhältnis von (Meth)acrlysäureanhydrid zu Amin beträgt zwischen 0,8:1,2 bis 1,2:0,8 , bevorzugt 1:1. Die Zudosierung der wässrigen Aminlösung zum (Meth)acrylsäureanhydrid erfolgt mit einer Geschwindigkeit, so dass die anfängliche Reaktionstemperatur der exothermen Reaktion nicht über 25 °C, bevorzugt nicht über 15 °C ansteigt. Die weitere Zudosierung ist so zu führen, dass die Reaktorinnentemperatur umsatzbezogen bis zu einem Maximalwert von 40 °C ansteigt und die Temperatur der Kühlflüssigkeit, die zur Kühlung des Reaktionsbehälters eingesetzt wird, zu keinem Zeitpunkt unterhalb der Kristallisationstemperatur des entstehenden Amids in der Reaktionslösung liegt. Der Reaktordruck ist hierbei zwischen 0,5 bis 6 bar, bevorzugt zwischen 0 und 1 bar zu halten.

Zur Neutralisation wird die Reaktionslösung in einen weiteren Behälter, bevorzugt mit Rührwerk ausgestattet, überführt. Die Neutralisation erfolgt mit alkalischen Lösungen, bevorzugt mit NaOH- oder KOH-Lösung, besonders bevorzugt mit Ammoniaklösung. Zuvor kann die Reaktionslösung optional mit Wasser bzw. der Zentrifugenmutterlauge verdünnt werden. Vor Beginn der Neutralisation wird die Reaktionslösung auf unter 20 °C, bevorzugt auf unter 15°C abgekühlt. Die Zuführung der alkalischen Lösung muss so erfolgen, dass zu keinem Zeitpunkt 60 °C, bevorzugt 50 °C überschritten werden. Nach Neutralisation wird der Ansatz mit den ausgefallenen Produktkristallen auf unter 10 °C, bevorzugt auf 5 °C, besonders bevorzugt auf -5 °C abgekühlt.

Zur Gewinnung des Feststoffs aus dem neutralisierten Ansatz können dem Fachmann vertraute fest/flüssig Trennmethoden wie z.B. Filtrieren oder Zentrifugieren eingesetzt werden. Im erfindungsgemäßen Verfahren wird Zentrifugieren mit einem optional vorgeschalteten Mahlschritt bevorzugt. Zum Mahlen der Kristallsuspension können z.B. Suspensions-Mühlen bzw. Nassmühlen (z.B. Fa. Fryma), eingesetzt werden. Das Zentrifugieren erfolgt bei Temperaturen unter 10 °C, bevorzugt unter 5 °C, insbesondere unter -5 °C. Die Ausbeute an N-Akyl(meth)acrylamid liegt normalerweise zwischen 80 und 85 % bezogen auf N-Alkyl(meth)acrylanhydrid.

Das nachfolgende Beispiel soll das erfindungsgemäße Verfahren erläutern, aber in keiner Weise einschränken.

### Beispiel 1:

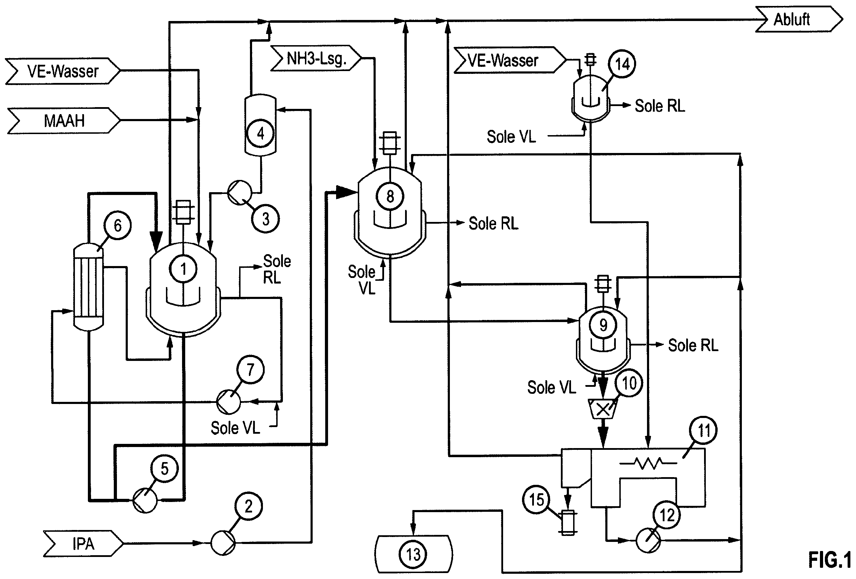

Die Herstellung von N-Isopropylmethacrylamid (NIPMAA) erfolgt nach Abb. 1. 800 kg Methacrylsäureanhydrid werden im Rührreaktor (1) vorgelegt und nach Versetzen mit 135 g 4-Methyl-2,6-di-tert.-butylphenol und 115 g 4-Hydroxy-2,2,6,6-tetramethylpiperidin-1-oxyl auf -5 °C vorgekühlt. Der Reaktor (1) wird mit Stickstoff gespült. Die Druckhaltung wird auf 1,3 bar eingestellt. 437 kg 70 %ige Isopropylaminlösung werden über die Pumpe (2) in die Vorlage (4), die gekühlt werden kann, gepumpt. Via Dosierpumpe (3) wird das Isopropylamin in den Reaktor (1) zudosiert, so dass die Reaktionstemperatur während der Reaktion zwischen -5 und 40 °C liegt. Während der Reaktion wird der Reaktorinhalt mit der Pumpe (5) über den Wärmetauscher (6) umgewälzt. Die Reaktionswärme wird durch die Solekühlung (7) des Reaktors (1) und des Wärmetauschers (6) abgeführt. Nach erfolgter Zugabe der Isopropylaminmenge erfolgt eine Nachreaktion von 15 min bei 20-40 °C, bevorzugt bei 35-40 °C. Die nach der Reaktion entstehende Aufschlämmung von NIPMAA in Methacrylsäure wird zur Viskositätsminderung mit 1100 kg VE-Wasser (demineralisiertes Wasser) verdünnt. Der verdünnte Ansatz wird mit der Pumpe (5) in den Rührbehälter (8) überführt und mit 355 kg 25 %iger Ammoniaklösung unter Rühren neutralisiert. Bei der Neutralisation fällt das NIPMAA zum Großteil aus, das gebildete Ammoniummethacrylat bleibt in Lösung. Zur NIPMAA-Abtrennung wird die Suspension über die Puffervorlage (9) abgelassen, welche zur Pufferung der Kristallmaische verwendet wird. Die Puffervorlage (9) ist gekühlt (bis max. -5 °C). Die gepufferte Suspension wird über die Mühle (10) auf die Zentrifuge (11) gegeben. Über den VE-Wasserbehälter (14) wird kaltes VE-Wasser (∼5 °C) bereitgestellt, welches zum Waschen der Kristalle in der Zentrifuge (11) verwendet wird, um die notwendige Reinheit zu erhalten. Die wässrige Mutterlauge, die neben NIPMAA hauptsächlich Ammoniummethacrylat enthält, wird mit Pumpe (12) in (8) oder (9) zurückgepumpt. Am Ende der Zentrifugation wird die Mutterlauge komplett in Behälter (13) gesammelt. Die Ausbeute an NIPMAA in der NIPMAA-Vorlage (15) beträgt 84 % bezogen auf Methacrylsäureanhydrid.

Wird der Ansatz in der Reaktionsphase zu stark gekühlt, entstehen Kristalle und Beläge an den Reaktorwänden und im Wärmetauscher, wonach die Reaktion abgebrochen werden muss. Übersteigt im Gegensatz dazu die Reaktionstemperatur die angegebenen Grenzen bilden sich qualitätsmindernde Verfärbungen und Ausbeuteverluste. Ähnliche Probleme entstehen, wenn das entsprechende Amin vorgelegt und (Meth)acrylsäureanhydrid zudosiert wird.

Die nach dem erfindungsgemäßen Verfahren hergestellten N-Alkyl(meth)acrylamide können in an sich bekannter Weise zu Polymeren und Copolymeren zur Verwendung in vielfältigsten Anwendungen umgesetzt werden.

**Bezugszeichenliste:**

| Nummer | Bezeichnung |
|---|---|
| 1 | Rührreaktor |
| 2 | Pumpe |
| 3 | Dosierpumpe |
| 4 | Vorlagebehälter |
| 5 | Pumpe |
| 6 | Wärmetauscher |
| 7 | Solekühlung |
| 8 | Rührbehälter |
| 9 | Puffervorlage |
| 10 | Mühle |
| 11 | Zentrifuge |
| 12 | Pumpe |
| 13 | Behälter |
| 14 | VE-Wasser Behälter |
| 15 | NIPMAA-Vorlage |

## Patentansprüche

1. Verfahren zur Herstellung von N-Alkyl(meth)acrylamiden der allgemeinen Formel (1), 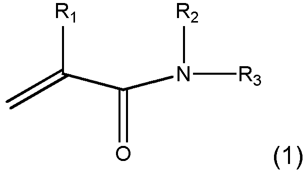 wobei R₁ für eine H- oder CH₃-Gruppe, R₂ und R₃ für Wasserstoff oder einen linearen, verzweigten oder cyclischen Alkylrest oder Arylrest mit 1 bis 12 C-Atomen, sowie Reaktionsprodukte von (Meth)acrylsäureanhydrid mit Aminen aus der Gruppe primärer substituierter aliphatischer Amine, Benzylamin, Allylamin, isomerer Aminotoluole, einzeln oder in Mischungen, isomerer Xylidine, einzeln oder in Mischungen, die durch ein oder mehrere Halogene substituiert sind, sekundärer aliphatischer, cycloaliphatischer oder aromatischer Amine, die durch ein oder mehrere Halogene substituiert sind, Dibenzylamin, Morpholin, substituierter Piperidine stehen
**dadurch gekennzeichnet, dass**
man vorgelegtes (Meth)acrylsäureanhydrid mit einer wässrigen Lösung eines Alkylamins der Formel (2),
R₂R₃NH (2)
wobei R₂ und R₃ für Wasserstoff oder einen linearen, verzweigten oder cyclischen Alkylrest oder Arylrest mit 1 bis 12 C-Atomen, sowie Amine aus der Gruppe primärer substituierter aliphatischer Amine, Benzylamin, Allylamin, isomerer Aminotoluole, einzeln oder in Mischungen, isomerer Xylidine, einzeln oder in Mischungen, die durch ein oder mehrere Halogene substituiert sind, sekundärer aliphatischer, cycloaliphatischer oder aromatischer Amine, die durch ein oder mehrere Halogene substituiert sind, Dibenzylamin, Morpholin, substituierter Piperidine stehen
umsetzt und das entstehende Amid abtrennt,
die Umsetzung bei -5°C bis 40°C bei einem Druck von 0,5 bis 6 bar, bevorzugt bei 0 bis 1 bar, erfolgt,
wobei die Zugabe der wässrigen Aminlösung zum (Meth)acrylsäureanhydrid mit einer Geschwindigkeit erfolgt, dass die anfängliche Reaktionstemperatur der exothermen Reaktion nicht über 25°C ansteigt,
und die weitere Zudosierung so erfolgt, dass die Reaktorinnentemperatur umsatzbezogen bis zu einem Maximalwert von 40°C ansteigt
und
die Temperatur der Kühlflüssigkeit, die zur Kühlung des Reaktionsbehälters eingesetzt wird, zu keinem Zeitpunkt unterhalb der Kristallisationstemperatur des N-Alkyl(meth)acrylamids in der Reaktionslösung liegt.

2. Verfahren zur Herstellung eines N-Alkyl(meth)acrylamids gemäß Anspruch 1,
**dadurch gekennzeichnet, dass** Vorlage und Alkylamin vor der Reaktion auf unterhalb 30 °C, bevorzugt auf 10 bis -5 °C vorgekühlt werden.

3. Verfahren zur Herstellung eines N-Alkyl(meth)acrylamids gemäß Anspruch 1,
**dadurch gekennzeichnet, dass** man die entstehende Lösung aus N-Alkyl(meth)acrylamid und (Meth)acrylsäure mit Wasser, optional mit der Zentrifugen-Mutterlauge, verdünnt, anschließend mit alkalischer Lösung, bevorzugt Ammoniaklösung, NaOH oder KOH neutralisiert und das ausfallende N-Alkyl(meth)acrylamid abtrennt.

4. Verfahren zur Herstellung eines N-Alkyl(meth)acrylamids gemäß Anspruch 1,
**dadurch gekennzeichnet, dass** Reaktion sowie Neutralisation und Fällung jeweils in einem getrennten Behälter erfolgen.

5. Verfahren zur Herstellung eines N-Alkyl(meth)acrylamids gemäß Anspruch 1,
**dadurch gekennzeichnet, dass** das in der Reaktion umzusetzende Alkylamin in einer 50-90 %igen, bevorzugt in einer 60-80 %igen, besonders bevorzugt in einer 65-75 %igen wässrigen Lösung, dem (Meth)acrylsäureanhydrid zugesetzt wird.

6. Verfahren zur Herstellung eines N-Alkyl(meth)acrylamids gemäß Anspruch 1,
**dadurch gekennzeichnet, dass** die Umsetzung in Anwesenheit von wasserlöslichen Polymerisationsinhibitoren erfolgt, die unter Inertgas, bevorzugt Stickstoff, wirken.

7. Verfahren zur Herstellung eines N-Alkyl(meth)acrylamids gemäß Anspruch 1,
**dadurch gekennzeichnet, dass** die Produktkristallmaische vor der Abtrennung in der Zentrifuge in einer Mühle gemahlen wird.

8. Verfahren zur Herstellung eines N-Alkyl(meth)acrylamids gemäß Anspruch 1,
**dadurch gekennzeichnet, dass** der Reaktor mit einem aussenliegenden Wärmetauscher und einer Zirkulationspumpe ausgerüstet ist.

## Claims

1. Process for preparing N-alkyl(meth)acrylamides of the general formula (1), 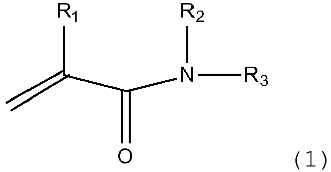 where R₁ is H or a CH₃ group, R₂ and R₃ are hydrogen or a linear, branched or cyclic alkyl radical or aryl radical having from 1 to 12 carbon atoms and also reaction products of (meth)acrylic anhydride with amines from the group consisting of primary substituted aliphatic amines, benzylamine, allylamine, isomeric aminotoluenes, individually or in mixtures, isomeric xylidines, individually or in mixtures, which are substituted by one or more halogens, secondary aliphatic, cycloaliphatic or aromatic amines which are substituted by one or more halogens, dibenzylamine, morpholine, substituted piperidine,
**characterized in that**
initially charged (meth)acrylic anhydride is reacted with an aqueous solution of an alkylamine of the formula (2),
R₂R₃NH (2)
where R₂ and R₃ are hydrogen or a linear, branched or cyclic alkyl radical or aryl radical having from 1 to 12 carbon atoms, or else amines from the group consisting of primary substituted aliphatic amines, benzylamine, allylamine, isomeric aminotoluenes, individually or in mixtures, isomeric xylidines, individually or in mixtures, which are substituted by one or more halogens, secondary aliphatic, cycloaliphatic or aromatic amines which are substituted by one or more halogens, dibenzylamine, morpholine, substituted piperidines, and the amide formed is separated off,
the reaction is carried out at from -5°C to 40°C at a pressure of from 0.5 to 6 bar,
preferably at from 0 to 1 bar,
where the addition of the aqueous amine solution to the (meth)acrylic anhydride is carried out at such a rate that the initial reaction temperature of the exothermic reaction does not rise above 25°C,
and the further introduction is carried out in such a way that the internal reactor temperature rises, as a result of the reaction, to a maximum value of 40°C
and
the temperature of the cooling liquid used for cooling the reaction vessel is not below the crystallization temperature of the N-alkyl(meth)acrylamide in the reaction solution at any point in time.

2. Process for preparing an N-alkyl(meth)acrylamide according to Claim 1, **characterized in that** reservoir and alkylamine are precooled to below 30°C, preferably to from 10 to -5°C, before the reaction.

3. Process for preparing an N-alkyl(meth)acrylamide according to Claim 1, **characterized in that** the resulting solution of N-alkyl(meth)acrylamide and (meth)acrylic acid is diluted with water, optionally with the centrifuge mother liquor, subsequently neutralized by means of an alkaline solution, preferably ammonia solution, NaOH or KOH, and the precipitated N-alkyl(meth)acrylamide is separated off.

4. Process for preparing an N-alkyl(meth)acrylamide according to Claim 1, **characterized in that** the reaction and also the neutralization and precipitation are each carried out in a separate vessel.

5. Process for preparing an N-alkyl(meth)acrylamide according to Claim 1, **characterized in that** the alkylamine to be reacted in the reaction is added in a 50-90% strength, preferably 60-80% strength, particularly preferably 65-75% strength, aqueous solution to the (meth)acrylic anhydride.

6. Process for preparing an N-alkyl(meth)acrylamide according to Claim 1, **characterized in that** the reaction is carried out in the presence of watersoluble polymerization inhibitors which act under inert gas, preferably nitrogen.

7. Process for preparing an N-alkyl(meth)acrylamide according to Claim 1, **characterized in that** the product crystal slurry is milled in a mill before being separated off in the centrifuge.

8. Process for preparing an N-alkyl(meth)acrylamide according to Claim 1, **characterized in that** the reactor is equipped with an external heat exchanger and a circulation pump.

## Revendications

1. Procédé de fabrication de N-alkyl(méth)acrylamides de formule générale (1) 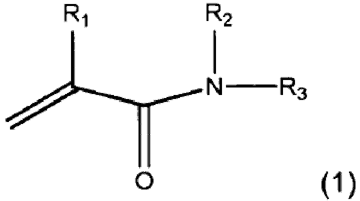 dans laquelle R₁ représente un groupe H ou CH₃, R₂ et R₃ représentent l'hydrogène ou un radical alkyle linéaire, ramifié ou cyclique ou un radical aryle de 1 à 12 atomes C, ainsi que de produits de réaction d'anhydride de l'acide (méth)acrylique avec des amines du groupe constitué par les amines aliphatiques substituées primaires, la benzylamine, l'allylamine, les aminotoluènes isomères, individuellement ou en mélanges, les xylidines isomères, individuellement ou en mélanges, qui sont substituées par un ou plusieurs halogènes, les amines aliphatiques, cycloaliphatiques ou aromatiques secondaires, qui sont substituées par un ou plusieurs halogènes, la dibenzylamine, la morpholine, les pipéridines substituées,
**caractérisé en ce que**
l'anhydride de l'acide (méth)acrylique chargé initialement est mis en réaction avec une solution aqueuse d'une alkylamine de formule (2)
R₂R₃NH (2)
dans laquelle R₂ et R₃ représentent l'hydrogène ou un radical alkyle linéaire, ramifié ou cyclique ou un radical aryle de 1 à 12 atomes C, ainsi des amines du groupe constitué par les amines aliphatiques substituées primaires, la benzylamine, l'allylamine, les aminotoluènes isomères, individuellement ou en mélanges, les xylidines isomères, individuellement ou en mélanges, qui sont substituées par un ou plusieurs halogènes, les amines aliphatiques, cycloaliphatiques ou aromatiques secondaires, qui sont substituées par un ou plusieurs halogène, la dibenzylamine, la morpholine, les pipéridines substituées,
et l'amide formé est séparé,
la réaction ayant lieu à une température de -5 °C à 40 °C à une pression de 0,5 à 6 bar, de préférence de 0 à 1 bar, l'ajout de la solution aqueuse d'amine à l'anhydride de l'acide (méth)acrylique ayant lieu à une vitesse telle que la température de réaction initiale de la réaction exothermique n'augmente pas au-dessus de 25 °C,
et l'ajout ultérieur ayant lieu de telle sorte que la température intérieure du réacteur augmente en fonction de la conversion jusqu'à une valeur maximale de 40 °C,
et
la température du liquide réfrigérant qui est utilisé pour le refroidissement du contenant de réaction ne se situant à aucun moment en dessous de la température de cristallisation du N-alkyl(méth)acrylamide dans la solution de réaction.

2. Procédé de fabrication d'un N-alkyl(méth)acrylamide selon la revendication 1, **caractérisé en ce que** la charge initiale et l'alkylamine sont prérefroidies avant la réaction à une température inférieure à 30 °C, de préférence de 10 à -5 °C.

3. Procédé de fabrication d'un N-alkyl(méth)acrylamide selon la revendication 1, **caractérisé en ce que** la solution formée de N-alkyl(méth)acrylamide et d'acide (méth)acrylique est diluée avec de l'eau, éventuellement avec la liqueur mère de centrifugation, puis neutralisée avec une solution alcaline, de préférence une solution d'ammoniac, NaOH ou KOH, et le N-alkyl(méth)acrylamide précipité est séparé.

4. Procédé de fabrication d'un N-alkyl(méth)acrylamide selon la revendication 1, **caractérisé en ce que** la réaction, ainsi que la neutralisation et la précipitation ont chacune lieu dans un contenant séparé.

5. Procédé de fabrication d'un N-alkyl(méth)acrylamide selon la revendication 1, **caractérisé en ce que** l'alkylamine à mettre en réaction dans la réaction est ajoutée à l'anhydride de l'acide (méth)acrylique dans une solution aqueuse à 50 à 90 %, de préférence 60 à 80 %, de manière particulièrement préférée 65 à 75 %.

6. Procédé de fabrication d'un N-alkyl(méth)acrylamide selon la revendication 1, **caractérisé en ce que** la réaction a lieu en présence d'inhibiteurs de polymérisation solubles dans l'eau qui agissent sous gaz inerte, de préférence azote.

7. Procédé de fabrication d'un N-alkyl(méth)acrylamide selon la revendication 1, **caractérisé en ce que** la suspension de cristaux de produit est broyée dans un broyeur avant la séparation dans la centrifugeuse.

8. Procédé de fabrication d'un N-alkyl(méth)acrylamide selon la revendication 1, **caractérisé en ce que** le réacteur est équipé d'un échangeur de chaleur extérieur et d'une pompe de circulation.
